# EUROPEAN PATENT SPECIFICATION

(11) **EP 1 844 725 B1**
(45) Date of publication and mention of the grant of the patent: **25.03.2009**
(21) Application number: 06007425.9
(22) Date of filing: 07.04.2006
(51) Int. Cl.: A61B 19/00, G06F 19/00

(54) **Risk assessment for planned trajectories**
Risikobewertung von geplanten Trajektorien
Évaluation des risques de trajectoires planifiés

(43) Date of publication of application: 17.10.2007
(73) Proprietor: BrainLAB AG, 85622 Feldkirchen (DE)
(72) Inventor: Hartlep, Andreas, 83629 Weyarn (DE); Pedain, Christoph, 81667 München (DE); Rodriguez Ponce, Maria Inmaculada, 81669 München (DE)
(74) Representative: Schwabe - Sandmair - Marx

(56) References cited:
- EP-A- 0 945 815
- EP-A- 1 396 233
- US-A1- 2003 114 751
- US-A1- 2003 208 116
- US-A1- 2004 015 070
- US-A1- 2004 111 183
- US-A1- 2006 052 689

## Description

The present invention relates to methods, devices and computer programs which are used for preparing and planning the placement of catheters, especially intra-cranial catheters, in or into a body and according to a specific embodiment also for carrying out an infusion, drainage of a substance, such as fluid, into or out of tissue, or biopsies, preferably after having prepared and planned the placement of the device.

In general, the term "infusion" is to be understood in accordance with the invention as any administering of for example a liquid or solid substance and/or an infusing medium such as for example pharmaceutical substances, cells, genes, enzymes, proteins, liposomes, antibodies, hormones, viral vectors, viruses or the like, said substance being introduced directly into a body and/or into body tissues, in order for example to reach a pre-defined target area and/or to bypass the effect of the blood-brain barrier. The substance can be delivered within a relatively short period of time, for example through an injection, or over a longer period of time, for example at a continuous and possibly variable rate of delivery of the substance.

Pharmaceutical substances have previously been administered by injecting a substance through the skin, directly into the vascular system, muscle tissue or subcutaneous tissue, or by positioning a catheter such that a substance could be introduced from directly into the targeted body tissues. This depends crucially on the experience of the person who for example positioned the syringe or catheter so as to be able to position the substance to be administered as precisely as possible in the desired area of tissue. Furthermore, the behaviour of said substance in the specific area of tissue must be known and taken into consideration to ensure that the resulting distribution of the substance matches the desired target area. Due to intrinsic and unavoidable inaccuracies related to the planning and placement procedure (e.g. from image resolution registration accuracy, etc.) and to different levels of experience of the executing physician and/or the lack of knowledge of the patient-specific tissue configuration, as well as due to patient-specific variations in the arrangement of the tissue, for example in the case of a diseased tissue, it has been necessary to leave the sufficient space between critical areas (in terms of a specific riskiness, automatically and/or manually defined), e.g. eloquent brain areas, vascular structures or anatomical areas such as e.g. ventricles or the visual nerve, and the actual trajectory, in order to ensure that the catheter does not interfere with the critical area.

Thus, although there might be a trajectory which could be used because it avoids all non-desired areas and structures, this trajectory might not be chosen due to the close proximity to such critical area. Therefore, the catheter is not placed as close as possible next to an area to be treated to avoid the risk of injuring a critical area.

On the other hand, it must be ensured, when performing an infusion, that the substance distribution resulting from the infusion optimally matches the desired target area, which could necessitate, in case of suboptimal catheter placement, that, e.g. a larger amount of a toxic substance for treatment has to be infused than would have been necessary if the catheter could be placed in close proximity or directly into the area to be treated.

According to the prior art, trajectories for biopsies or intra-cranial catheters are planned prior to neurosurgery. The planning is based on target selection and on a selection of the entry point of the trajectory. To perform a minimal invasive surgical procedure, the physician plans the trajectory in consideration of critical brain areas. These areas might consist of critical and anatomical structures, such as ventricles, or physiological, vascular or functional structures.

Various methods and devices have already been proposed, for enabling a substance to be introduced into a particular area of tissue in the first place, or for developing this to be more efficient, for example for treating tumours, Parkinson's disease or other diseases.

It is known from US RE 37,410 E to inject a substance to be administered into a bio-degradable material and to arrange the latter within or closely adjacent to a tumour to be treated, in order for example to bypass the effect of the blood-brain barrier in the case of a brain tumour. Once the bio-degradable material has degraded, the substance contained in it is released. This delivery method based on diffusive processes, however, is relatively imprecise with respect to individual, patient-specific dosage.

US 6,026,316 describes a method for delivering medicines, using data obtained from magnetic resonance imaging (MRI) to determine the position of the delivery device and to monitor the spatial distribution of the delivered medicine.

US-2003-01-147051-A1 discloses a method for planning an infusion, wherein patient data is captured and the infusion to be carried out is planned using this patient data.

US 2004/0015070 A1 discloses computer aided treatment planning and discloses a system that provides warnings to a user if a proposed intervention presents risk of damage to surrounding structures. The virtual intervention includes the placement of a virtual cylinder and a warning can be provided if the proximity of the virtual cylinder to anatomical structures of the ear is less than a predetermined threshold distance. A warning can also be provided if the proposed path of a biopsy needle will damage anatomical structures in the vicinity. A warning can further be provided if the generalized virtual cylinder placement will damage critical anatomical structures. The virtual cylinder may be a deformable model when the tool being modeled is a flexible catheter.

It is an object of the present invention to propose methods, devices and software using which a device can be safely introduced into a body and moved in the body.

This object is solved by the subject of the independent claims. Advantageous embodiments follow from the dependent claims.

According to a first aspect, the invention relates to a method for planning the placement of a device into a body or the movement of the device in the body, wherein at least a part of the internal structure or the whole internal structure of the body is analyzed to determine, if a critical region of the body such as a critical anatomical structure, as e.g. ventricles, or physiological or functional structure, which should preferably not be harmed, is in or near the planned trajectory of the device in the body or within a region of interest around the planned trajectory. The device can be any device used for medical examination or treatment and can be for example an intra-cranial catheter, which should be safely introduced and placed into the body and moved therein without harming the patient, e.g. by crossing an optical nerve or another important anatomical or functional risk structure, while simultaneously considering e.g. the success of a medical examination or therapy by reaching a specific body structure, e.g. by covering a target area with a substance distribution resulting from the location and trajectory of the catheter. According to the invention, the levels of risk e.g. resulting from a specifically planned trajectory or the difficulty in carrying out a surgical plan or medical examination associated with planned stereotactic trajectories can be assessed and related to a specific plan or trajectory of a device to be introduced into, placed in and/or moved in the body or patient. Especially, when considering a particular plan for placing a device in a body, the proximity of a treatment approach, e.g. a trajectory of the device, to risk structures can be determined and the riskiness of a particular treatment approach can be weighted, so that for example a plan can be drawn up that the catheter to be placed in the body has a predetermined minimum distance from certain critical structures and stays e.g. at least 5 mm away from sulci or 7 mm away from the optical nerve.

The three dimensional structure of the body or tissue can be considered and inaccuracies of the planning and placement approach, which can probably result e.g. from the restricted image resolution, the non-perfect patient registration, the instability or flexibility of a catheter and so on, can be determined or anticipated for consideration, so that the risk of reaching or not reaching a specific structure can be rated. For example, it can be considered that a catheter having a high flexibility may be deflected to cross a critical region, which may increase the determined level of risk. Furthermore, it can for example be easy to stay away from a critical area, such as a sulcus, by a predetermined distance of for example 5 mm at the point of entry, whereas further down the trajectory, i.e. in the body or patient, the predetermined distance of e.g. 5 mm is not sufficient if for example an instable catheter is used which is probably bent toward the risk structure.

Furthermore, inaccuracy based on the navigational approach, such as frame based or frameless, can be considered for planning the trajectory to guarantee that a specific structure is or is not reached by introducing the device. Image resolution and data accuracy can be taken into consideration to determine the accuracy of the method and thus the risk of reaching risk structures.

According to the invention the risk due to a particular setup or configuration of a surgical navigation system can be considered. For delivering a probe, the holding device used in surgery bears inherent inaccuracies, e.g. if a surgeon uses a pointer to pass a catheter along, the anticipated inaccuracies are higher than with a rigidly mounted holding device. Also, if the holding device is fixed far away from the entry point, the risk of inaccurate placement is elevated versus fixing the holding device close to the entry point, which inaccuracies can be determined or estimated according to the invention and can be used for planning the movement of a device into or in a body or patient. A catheter having a high flexibility may be deflected into or towards a structure of risk, which would increase the respective level of risk, whereas a stiff or hard catheter may decrease this level of risk, whereas a stiff catheter would rise the risk of e.g. harming vascular structures which is less likely for a more flexible catheter. Also an experienced or a good surgeon may decrease the calculated level of risk, whereas an inexperienced surgeon may increase it.

Thus, according to the invention, the structures of the body or patient or a part of the body, such as the brain, can be detected automatically to detect the anatomical, functional and/or physiological structures by using e.g. known segmentation techniques. Furthermore, it is also possible to use manually outlined structures which can be identified e.g. by an experienced surgeon. These structures can be manually or automatically related to different levels of risk.

The analysis of the body or a part thereof, especially structural data, can be performed on the basis of anatomical data obtained by e.g. MRI, CT, X-Ray, ultrasound and / or physiological data obtained by MR-DTI, MR-DCE, perfusion imaging from MR or CT, PET, SPECT, MEG and / or functional data, obtained by fMRI, PET, brain-mapping, EEG and/or angiographic data which is able to visualize veins and/or arteries.

The method of the invention is able to either detect automatically anatomical, functional, angiographic and/or physiological structures of the body, especially the brain and/or manually outlined structures. The structures can be identified in the internal structure of the body or in images and / or data of the internal structure of the body and can be segmented e.g. automatically from said images. These structures can either manually or automatically be related to different levels of risk.

After entering a trajectory e.g. for biopsy, catheter placement, shunt placement or stimulator placement based on the patient-specific input data and/or automatically and/or manually outlined structures, the method of the present invention can preferably check for existence of critical structures in a selected area around the trajectory. The volume or region of interest can be defined as a default value or by a predetermined value around or close to the trajectory or can be defined manually by the user, or can be automatically or semiautomatically defined using information about patients and/or treatments and or the treating physicians experience. Critical regions or structures appearing within this volume can be automatically displayed and/or classified in terms of levels of risk. Different levels of risk can be defined in terms of proximity or likelihood of reaching this structures or can be determined based on proximity to the trajectory, anatomical characteristics, functional characteristics as well as physiological characteristics of the critical structure. Also close proximity between different trajectories can be included as a risk. In general, these characteristics can be used to assess potential risk levels for affecting functionality or any adverse effect due to the crossing of the trajectory in the respective region. Information of the identified critical structure can e.g. be displayed in terms of:
- Spatial Location
- Level of risk / potential adverse effect
- Proximity to the trajectory

Regarding to the spatial location, the critical area can be obtained by patient-specific image data and/or can be outlined manually by the user. In one embodiment, the segmented critical structure can be registered with an image of the part of the body and might then be overlaid on the co-registered subject image. Levels of risk can be displayed e.g. on a separate window and/or by colour classification of the segmented critical structure. Warnings about the potential adverse effect e.g. from crossing this structure can be also displayed. Quantitative information regarding to the proximity of the critical structure with respect to one or more trajectories can also be provided.

An embodiment of the present invention is the use of the described information for navigation systems. Virtual "walls" or boarders can be created along or around the critical region that do not allow the user or warn the user if areas behind those walls or boarders are entered.

In another embodiment suggestions for trajectories that do (do not) cross regions of predefined or predetermined risk levels are created or determined. For example, the trajectory can be planned such that it only crosses areas or locations of a predetermined risk level but does not cross areas with a higher risk level. Furthermore, the distance from said areas which are not to be crossed by the trajectory can be related to or proportional to the height or extent of said risk level.

Another embodiment is the use of information for creation of suggestions for trajectories that are placed in a predefined distance to areas of predefined risk levels. This distance can be the same constant distance for every critical structure with a risk level above a predetermined risk level or can be related to or proportional to the extent of each level of risk.

Another embodiment is the provision of haptic feedback in a robotic or assisting arm system, whereby the feedback is based upon the proximity between a surgical tool moving in a known relationship to the robotic arm and regions of risk. For example, the distance of a surgical tool to critical structures can be determined repeatedly. Based on said distance and the risk level of each critical region, a strong or weak haptic feedback can be provided to make the robotic or assisting system change the direction or position of the surgical tool.

Another embodiment is the directed delivery of electrical energy, e.g. by neurostimulation, based on the knowledge of the proximity of a delivery device to risk structures.

Another embodiment is the directed delivery of a therapeutic agent based on the knowledge of the flow patterns into nearby structures of risk

The present invention allows the physician to focus on finding the optimal trajectory for a treatment or medical examination by providing all required information in terms of anatomical, functional and/or physiological information and/or based on warnings about the potential adverse effects when chosen trajectories cross a critical area or region as outlined. Transferring the information to a surgical navigation system increases safety for the patient by excluding areas associated to high-risk levels.

In accordance with another aspect, the invention relates to a method for planning an infusion, i.e. for administering a substance or an active agent, in particular for injection into tissue, preferably into a predetermined tissue structure or tissue volume, wherein patient data or patient parameters obtained from them are captured. Known magnetic resonance imaging methods (MRI), computer tomography (CT) methods, x-ray methods, ultrasound methods or other suitable methods, which enable the spatial structure of a body, in particular of a tissue structure, to be detected and displayed and/or functional data, such as for example patient-specific diffusion and perfusion properties, to be obtained, can be used in this respect. Infusion is planned in accordance with the invention as described above using the patient data determined, i.e. for example the catheter to be used is suitably selected, the catheter is positioned with respect to the insertion location and depth of penetration, the infusing medium is selected and if necessary modified, for example thinned, and the pressure gradient over time with which the infusing medium is to be delivered through the catheter is predetermined, by taking into account various selectable pre-set figures, such as for example patient data or also parameters of available substances to be administered, parameters of the catheters which may be used and possibly of a pump which may be used. The aim of this selecting and setting is to inject a defined quantity of the substance to be administered into a target tissue volume, in order to obtain a particular concentration there, wherein as little of the substance to be administered as possible is to be introduced into non-target tissue.

The patient data captured may be used to position the infusion device(s), for example one or more catheters, wherein it is determined from these patient data where exactly in the patient's body a tissue volume to be treated, for example brain tumour, is situated. Using this information, a suitable catheter can be selected, for example from an available data base, by an operator or fully automatically, and possibly modified by post-processing, for example trimming the length of the catheter, in accordance with application or patient specifications, for example with respect to the desired depth of penetration into the tissue or the exact planned position of the catheter. Furthermore, it can be determined where a suitable point for inserting the catheter is or how the catheter should be placed in the tissue, in order that the infusion debilitates as little healthy tissue and as much non healthy tissue as possible.

Known positioning methods can advantageously be used, for example using reflecting markers which are attached to the catheter and detected by IR cameras, in order to attach the catheter at a desired position on the patient. To this end, markers may also be attached to the patient himself/herself, which serve as a reference and through which a patient coordinate system is determined in which the catheter is placed at a particular, determined point.

Patient-specific parameters are preferably determined using the captured patient data for planning the infusion, for example the tissue or body structure in the area of the tissue to be treated by the infusion. It is particularly advantageous to determine the tissue density, the distribution of particular tissue structures, or the blood flow through a particular area of tissue, as patient parameters. Patient parameters may be obtained both directly from the captured patient data as well as from databases or from a combination of values stored in data bases together with the captured patient data. Thus, values which may be used as patient parameters for planning an infusion can be stored, for example values in data bases relating to usual blood flow through particular areas of tissue, the diffusion and perfusion behaviour of selected substances in the tissue under consideration, values relating to tissue behaviour after a known substance has been delivered, for example swelling of the tissue or metabolic reactions.

It is furthermore advantageous to determine parameters of the infusing medium, which characterise the substance to be administered or an active agent and, for example, define the physical, chemical and/or biological properties. Thus, information relating to the molecular or particle size of the substance to be administered, the rate of diffusion of this substance in a particular type of tissue, the metabolism and/or interaction of the substance with tissue due to metabolic processes, a diffusion coefficient known for the substance for the type of tissue to be treated or advantageous injection pressure or pressure gradient, an advantageous concentration, quantity or rate of delivery, whose magnitude of size is usually in the range µl/min, can be obtained from a data base. The parameters of the infusing medium listed by way of example can be used individually or in combination with other parameters for planning the infusion.

Catheter variables are used, i.e. variables specific to a catheter for planning the infusion, wherein various types of catheter could be provided to choose from, for example in a data base. Catheter parameters relevant to the infusion can be for example, the inner diameter of the catheter, surface finish, the material, in particular the rigidity of the catheter, the shape, the number and arrangement of outlets on the catheter or a known suitability of a particular type of catheter for a particular substance to be administered or a particular type of tissue or diseased tissue to be treated. In general, a number of catheters may also be used in accordance with the invention.

In accordance with the invention, a physiological fluid, a pharmaceutical substance, a solution containing cells, viruses, viral vectors, genes, enzymes, proteins, liposomes, hormones, antibodies or a combination of those can be used as the infusing medium.

By using the patient parameters, parameters of the infusing medium and/or catheter parameters cited above by way of example, individually or in combination, together with the captured patient data, an infusion to be performed can be planned in accordance with the invention, such that as large a proportion of a substance as possible is introduced into a target area of tissue by infusion, wherein as little of the substance as possible is released into non-target tissue. Thus, in accordance with the invention, a substance to be introduced into a tissue by infusion can be introduced into an area of tissue to be treated in the patient using a particularly suitable and correctly positioned type of catheter and the correct injection pressure, at a suitable concentration and at a desired rate, taking into account metabolic and convective and/or diffusive and/or perfusive processes, in order to obtain a desired concentration of the substance to be introduced in said area of tissue, wherein - for example in the case of brain tumours - the effect of the blood-brain barrier may be bypassed by directly injecting the substance by infusion. Surrounding tissue is thus debilitated as little as possible.

In order to pre-plan the infusion, a simulation of the infusion to be performed can advantageously be calculated in accordance with the invention, for example by calculating the distribution of infusing medium in the tissue using the captured patient data and the various parameters mentioned above. Using such a simulation, the distribution of the infused medium can be determined both statically and dynamically as a function of time, and advantageously displayed graphically. In this way, it can be established even before performing an infusion whether a desired concentration distribution of the substance to be introduced in the target tissue can be obtained, or whether parameters of the infusing medium, catheter parameters or position parameters possibly have to be altered, to ensure a more successful infusion.

Furthermore, retro- or inverse planning can also be performed in accordance with the invention, wherein for example treatment data defined by an operator are pre-set, such as for example the target volume to be treated, advantageously together with high-risk structures such as for example nerve tracts which should not be compromised by the infusion, levels of risk of regions of the tissue, and details of the type of tissue to be treated. In this way, either fully automatically or by interaction with the operator, for example by displaying a selection menu, the course of the infusion can be established, i.e. one or more types of catheter can be selected together with suitable infusing media, the arrangement(s) of catheters can be determined with respect to position and/or depth of penetration and the parameters of the infusing medium can be set, in order to enable a optimal infusion treatment for the given target volume.

The planning methods described above, in particular the selecting of individual parameters and the segmentation of critical regions of the tissue and the determination of levels of risk in said region, can be performed: fully automatically, for example using values stored in data bases; semi-automatically, for example by selections - still to be made by an operator - from a displayed menu; or manually, for example through parameter values inputted by an operator. In this respect, suitable computers are advantageously used, together with input and output elements, for example display elements displaying elements to be selected, tissue structures, calculated concentration distributions of the infusing medium and other information.

In accordance with another aspect, the present invention relates to a computer program which, when loaded or running on a computer, performs the method described above or parts of it. Equally, the present invention relates to a storage medium for such a program or to a computer program product comprising the aforementioned program.

A device in accordance with the invention, for planning an infusion, comprises a planning system consisting of a computer system, preferably having input and output devices and corresponding software. In this respect, a monitor is advantageously provided for displaying elements pre-set by the computer from data bases or values determined from calculations or spatial distributions.

A navigation system is also advantageously provided, consisting for example of reflecting markers, LEDs or coils attached to elements to be positioned and IR cameras or magnetic field generators, with which a catheter on a body, for example, can be positioned using a suitable, known software and hardware.

Generally, the device in accordance with the invention includes elements, devices and systems with which the steps of the method described above may be performed.

Advantageously, verification is performed continuously or at particular intervals in time during the infusion, the distribution of the infusing medium in the tissue during or after the infusion process being determined using a suitable data capture or representation system. Magnetic resonance imaging, x-ray based methods, or ultrasound methods, for example, may be used in this respect, wherein it may be advantageous to add a contrast medium to the infusing medium in order to be able to establish or measure the distribution of the infusing medium in the body tissue clearly.

Preferably, deviations between the actual distribution of the infusing medium in the tissue determined in the verification process and the planning data determined before or during the infusion are determined and preferably displayed. Advantageously, the infusion parameters are corrected, i.e. the chemical and/or physical composition and/or properties of the infusing medium is/are changed and/or the delivery is changed, for example the injection pressure or quantity delivered is changed, in order to be able to correct the deviation, determined during verification, from the planned distribution. If necessary, a catheter can also be repositioned or exchanged.

Advantageously, the deviation is verified and determined and the correction made in real time, such that the infusion can be performed regulated via a back-coupling, to obtain the desired successful infusion, i.e. to deliver the infusing medium to the given target area as desired.

A device can be used for carrying out an infusion method as described above, comprising a verification device for determining the spatial distribution of an infusing medium in a body, in particular in an area of tissue. The verification device can for example be a magnetic resonance or nuclear spin resonance, x-ray, or ultrasound system with which the infusing medium or its distribution and concentration in the tissue can be detected.

Advantageously, a computer system is preferably provided with a display device, to evaluate the determined spatial distribution of the infusing medium in the tissue, establish a deviation from a previously established infusion plan and possibly to automatically alter the infusion parameters or propose such a change to an operator, in order to modify the infusion such that it can be carried out as planned. To this end, systems can for example be provided using which the concentration of the infusing medium can be changed and/or the injection pressure or injection quantity can for example be altered by means of a pump, to obtain a distribution of the infusing medium in the tissue as previously planned. When a deviation from a given infusion plan is established during verification, the manner and magnitude of the change to the infusion parameters are advantageously determined using known action and function mechanisms. For example, the rate of delivery or the injection pressure is reduced when it is established that the infusing medium is spreading faster than predicted or is not being degraded by metabolic processes as quickly as expected.

The invention will now be described by way of preferred example embodiments. There is shown:
- Figure 1a: an image acquired and displayed using an embodiment of the method of the present application,
- Figure 1b: a further image acquired and displayed using an embodiment of the method of the present application,
- Figure 2: a schematic diagram of a method for planning and carrying out an infusion;
- Figure 3: a simplified flow diagram of an infusion performed in accordance with the invention; and
- Figure 4: a device which may be used when planning and carrying out an infusion.

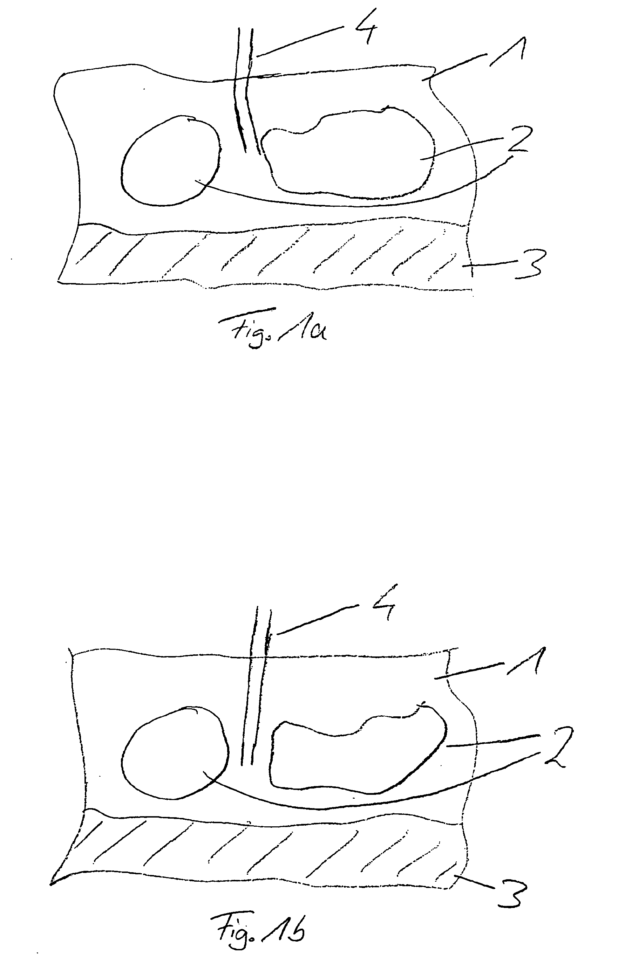

Figure 1a shows an image acquired using an embodiment of the method of the present invention for planning the movement and placement of a catheter 4 in tissue 1 of a patient having structures of risk 2, like damageable regions, and a target region 3 e.g. a region to be treated like a tumor. For efficiently treating the tumor 3 the catheter 4 should be positioned as near as possible to the tumor 3 or directly into the tumor 3 so that the substance can be directly injected into the tumor. The rest of the tissue should not come into contact with the substance and the catheter 4 should not cross an damageable area like the structures of risks 2 or should not be placed to close to said structures of risk 2. For this purpose an ultrasound imaging system images and displays the tissue 1, as shown in Figure 1a, so that the movement and placement of the catheter 4 can be planned and controlled. As shown in Figure 1a, a catheter 4 is inserted into the tissue 1 at a position suspected or determined to be the best position for inserting the catheter 4 for treating the tumor 3 without harming or damaging the structures of risk 2. However, as shown in Figure 1a, due to the internal structure of the tissue or due to the properties of the catheter, the catheter is bend towards a structure of risk 2. Further to displaying the tissue 1 and the movement of the catheter a warning device outputs a warning signal, when the catheter 4 comes closer to the structure of risk 2 then a predetermined or predefined safety range around the structure of risk 2. When the catheter crosses this safety range the warning device outputs a warning signal e.g. a sound signal or an image indicating danger or alarm like a yellow bar or a yellow sign. When the warning signal is ignored, i.e. by a surgeon and the catheter comes even closer to the structure of risk 2 the warning signal is increased, e.g. the loudness of the signal is increased or a sign indicating higher danger is displayed like a read bar or a red sign. Considering this warning signal, the catheter movement can be corrected or the catheter 4 can be pulled out of the tissue 1.

Figure 1b shows another attempt of positioning and moving the catheter such that the catheter 4 is directly inserted into the tumor 3 and the tumor can be ideally treated. By changing conditions or considering certain conditions, like changing the entry point of the catheter 4 or using a different catheter 4 like a more compact catheter or by considering the internal structure of the tissue 1 for determining a different trajectory or a different way for the catheter 4 to the tumor 3, the catheter 4 does not come to close to the structures of risk 2 and does not cross the safety region around the structures of risk 2, so that no warning signal is output by the warning device and the catheter can be perfectly inserted into the tumor 3.

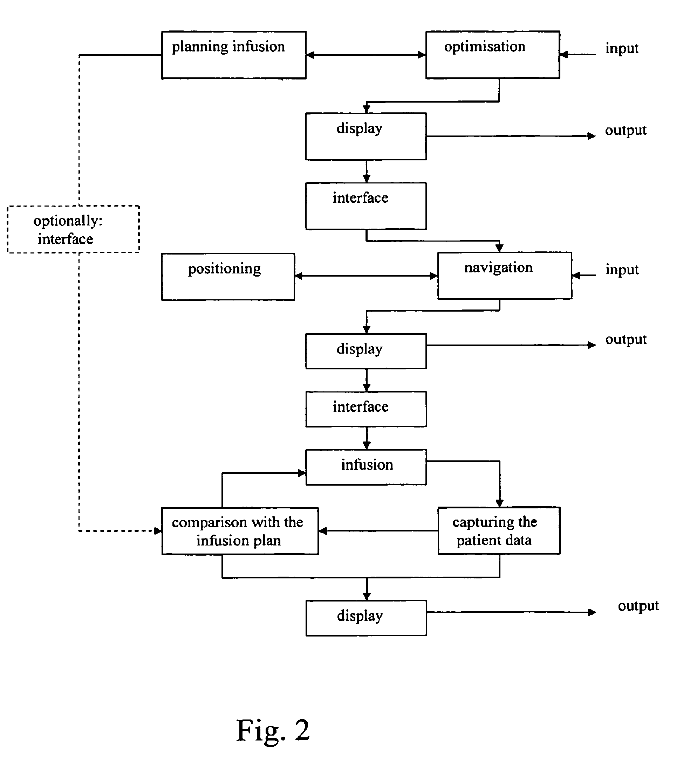

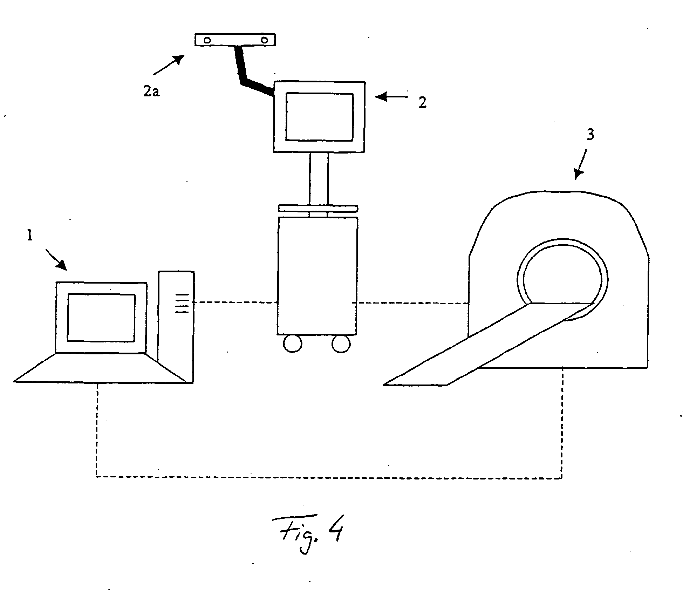

Figure 2 shows a schematic flow diagram for preparing and carrying out an infusion in accordance with the invention. As shown in Figure 2, patient data are inputted, for example from a magnetic resonance or nuclear spin tomograph, which are used to determine a particular target area of tissue for the infusion and to plan the infusion dosage to be delivered. These data can be obtained for example through the magnetic resonance or nuclear spin resonance system 3 as shown schematically in Figure 4, after a patient to be treated has been examined. Using parameters for the properties of the tissue structures, like data of levels of risk of critical structures, and for various types of catheter, stored for example in data bases, one or more catheters suitable for the infusion can be selected, once the exact location of the tissue volume to be treated has been determined. The patient parameters obtained, for example by the magnetic resonance or nuclear spin resonance method, can be used together with the catheter parameters and the parameters of the infusing medium, also for example stored in data bases, to plan the infusion. The corresponding parameters can thus be optimised, on the ancillary condition that a large as possible proportion of the infusing medium is introduced into the target tissue at the desired concentration, wherein as little of the infusing medium as possible is to reach tissue lying outside the target tissue. In general, as few catheters or needles should be placed as possible, said catheters or needles being fed through as few access ports as possible. This optimised planning of the infusion dosage is to be outputted via a display, such that for example a two-dimensional or three-dimensional representation can be outputted through representations of various incision planes, in order to display the results of infusion planning.

The infusion plan produced in this way is communicated via an interface to a navigation system, such as for example the VectorVision® system shown schematically in Figure 4, in order to position the selected catheter or catheters at the given points on the body, based on the planning data. The catheter(s) can be positioned automatically, for example using a robot, or by hand guided by the navigation system, a display device showing whether a catheter a correctly positioned or still has to be moved in a particular direction.

Once the catheter(s) has/have been successfully positioned, the actual infusion is carried out using the parameters of the infusing medium set by the planning. To this end, patient data are again captured, to determine the actual distribution of infusing medium in the tissue. Using the parameters set by the planning, and the results of the simulation of the infusion based on them, a comparison is made between the actual distribution of the infusing medium and the predicted, desired distribution of the infusing medium, and the parameters - for example the concentration of injecting medium, the quantity delivered or the injection pressure for carrying out the infusion - are altered as appropriate, preferably taking into account known action mechanisms in order to obtain the desired, planned result of infusion. The measured, actual distribution of the infusing medium concentration, preferably together with possible deviations and correcting methods, can again be outputted via a display, in order for example to enable an operator to manually intercede in the injection method.

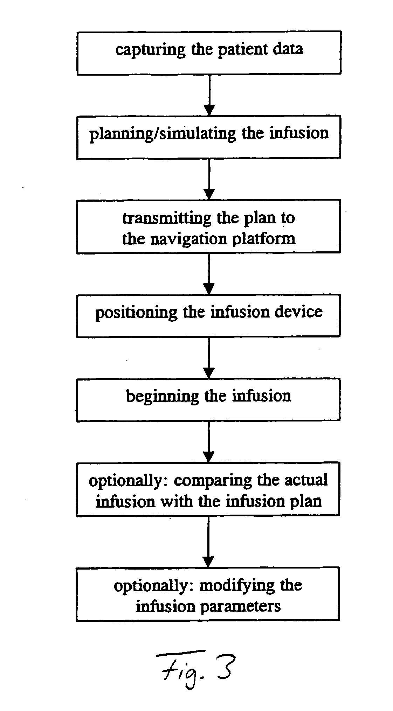

Figure 3 schematically shows a simplified sequence of planning and carrying out an injection in accordance with the invention. Firstly, patient data are captured using an imaging diagnostic method such as for example a magnetic resonance or nuclear spin resonance method, to obtain the current patient parameters such as for example tissue density, blood flow, and the location of a tissue to be treated. Using the patient parameters determined in this way, like data of levels of risk of critical regions, and with catheter parameters and parameters of the infusing medium obtained from a data base and/or pre-set for a particular infusion, the infusion is planned and/or simulated. Based on the parameter data determined in this way, the infusion plan is forwarded to a navigation platform, using which the catheter or catheters are to be positioned on the patient as provided for in the infusion plan. Infusion begins once the infusion device has been positioned and is carried out using the planned and possibly simulated parameters, wherein - as shown in Figures 2 and 3 - the infusion actually performed is optionally compared with the infusion plan, and in the event of deviations, the corresponding parameters are modified, preferably using known action mechanisms.

Figure 4 schematically shows a device, which may be used when planning and carrying out an infusion in accordance with the invention. Patient data are obtained in a magnetic resonance or nuclear spin tomograph 3 and forwarded to a planning system 1 and to a navigation system 2. The catheter or catheters are positioned at a desired point on a body by the navigation system 2, using for example known reflectors or markers, attached to one or more catheters, positional data of the markers being captured by IR cameras 2a. In order to carry out the infusion, the planning system 1 determines the suitable catheter parameters and parameters of the infusing medium for a pre-set infusion to be carried out, using patient parameters determined by the magnetic resonance or nuclear spin resonance system 3.

## Claims

1. Method for planning an infusion and the placement of a catheter having a specific rigidity chosen from various types of catherers provided in a database in a body, wherein information of at least a part of the internal structure of the body of a patient is automatically analyzed using segmentation techniques to determine if at least one specific or critical region or structure lies within a region of interest around the planned trajectory of the device in the body, wherein said specific or critical region or structure is automatically displayed and/or classified and/or assessed in terms of predefined, levels of risk using values stored in databases, wherein the levels of risk and the rigidity of the catheter are determined based on risk due to patient-specific tissue density of a specific region or structure of the body.

2. Method according to the previous claim, wherein as the information of at least a part of the internal structure of the body anatomical, functional, angiographical and/or physiological structures of the body are, particularly automatically, detected and/or manually outlined structures are detected.

3. Method according to any of the previous claims, wherein the anatomical structures are obtained using Magnetic Resonance Imaging (MRI), X-Ray Imaging, Computer Tomography (CT) Imaging, Ultra Sound Imaging, the physiological structures are determined using MR-DTI, MR-DCE, perfusion imaging from MR or CT, PET, SPECT, MEG, and the functional structures are determined using fMRI, PET, brain-mapping, EEG.

4. Method according to any of the previous claims, wherein the region of interest around the trajectory can be defined by at least one default or predetermined value close to the trajectory or around the trajectory and/or can be selected by a user and/or can be semiautomatically or automatically defined using information about the patient or the treatment of the patient.

5. Method according to any of the previous claims, wherein the specific or critical region or structure in the region of interest is further automatically displayed and/or classified and/or assessed in terms of spatial location, potential adverse effect and/or proximity to the trajectory.

6. Method according to any of the previous claims, wherein the specific or critical region or structure is identified in the information of at least a part of the internal structure of the body and is segmented from the information of the internal structure of the body.

7. Method according to any of the previous claims, wherein the specific or critical region or structure is obtained e.g. by MRI diffusion scans, MRI-contrast enhanced scans, DTI, MRI dynamic contrast enhanced scans, CT perfusion scans, MRI T1w scans, MRI T2w scans, MRI proton density scans, MRI spectroscopy scans, PET scans, SPECT scans, molecular imaging, CT, X-ray, ultrasound, elastography, time series imaging, biopsies and/or tissue analysis.

8. Method according to any of the previous claims, wherein the segmented specific or critical region or structure is registered with an image of the part of the internal structure of the body and is overlaid on said image.

9. Method according to the previous claim, wherein the specific or critical region or structure is displayed together with the planned trajectory on the image of the internal structure of the body.

10. Method according to any of the previous claims, wherein the levels of risk are determined based on:
- risk due to anatomical and/or functional and/ or physiological characteristics of a specific region or structure of the body, particularly risks of harming the body and/or risk of unsuccessful therapy; and/or
- risk due to a predefined treatment plan, particularly the proximity of a treatment approach, like a trajectory, to a risk structure, and/or the proximity of two or more different treatment approaches, like two or more trajectories, to each other and/or weighting of riskiness of the predefined treatment approach; and/or
- risk due to a predefined setup or configuration of a surgical navigation system

11. Method according to the previous claim, wherein the risk of harming the body comprises the risk of crossing a trajectory and an optical nerve and the risk of unsuccessful treatment comprises the risk of not crossing the trajectory through a Sulcus in drug delivery.

12. Method according to any of the two previous claims, wherein the weighting of riskiness of the predefined treatment approach comprises the weighting of riskiness of a catheter plan or trajectory which is at least a predetermined distance away from e.g. a sulcus.

13. Method according to any of the three previous claims, wherein the risk due to a predefined setup or configuration of the surgical navigation system comprises a risk of a use of an unmounted holding device, like a pointer for passing a catheter along the trajectory and/or the risk of a use of a flexible infusion or holding device and/or the risk when the holding device is fixed or mounted far away from an entry point of the catheter and/or the risk of an inexperienced surgeon.

14. Method according to any of the previous claims, wherein levels of risk are displayed separately and/or highlighted, e.g. colored, in the specific or critical region or structure.

15. Method according to any of the previous claims, wherein warnings about the potential adverse effect, like the crossing of the trajectory and the specific or critical region or structure, are displayed.

16. Method according to any of the previous claims, wherein quantitative information regarding to the proximity of the specific or critical region structure with respect to one or more trajectories is provided.

17. Method according to any of the previous claims, wherein borders are displayed along or around the specific or critical region or structure, wherein when planning the trajectory to cross at least one border, a warning signal, particularly an acoustic warning signal, is output or the trajectory is prevented to cross the border.

18. Method according to any of the previous claims, wherein trajectories are determined that do or do not cross regions of a predefined or predetermined level of risk, particularly which are placed in a predetermined distance to areas of a predefined or predetermined level of risk.

19. Method according to any of the previous claims, wherein the planned trajectory is used for stereotactic biopsies, catheter placement, cannula placement, stimulator placement, shunt placement, placement of surgical implants and/or placement of other devices.

20. Method according to any of the previous claims, wherein haptic feedback is provided to a robotic or semi-robotic device, particularly a robotic arm or a guiding device, whereby the haptic feedback is based upon the proximity between a surgical tool moving in a known relationship to the patient and regions of risk.

21. A computer program, which when running on a computer or loaded onto a computer, causes the computer to perform a method in accordance with any of the previous claims.

22. Computer program product stored on a computer-compatible medium or data carrier and comprising the computer program as set forth in the previous claim.

23. Device for planning an infusion and the placement of a catheter having a specific rigidity chosen from various types of catheters provided in a database in a body comprising:
- an acquisition device for acquiring information of at least a part of the internal structure of the body of a patient
- a database storing values for the determination of levels of risk
- an analyzing device for analyzing the acquired information of the internal structure of the body using segmentation techniques to determine if at least one specific or critical region or structure lies within a region of interest around the planned trajectory of the device in the body and to display and/or classify and/or assess said specific or critical region or structure in terms of predefined levels of risk using the values stored in the database, wherein the levels of risk and the rigidity of the catheter are determined based on risk due to patient-specific tissue density of a specific region or structure of the body.

24. Device according to claim 23, further comprising
a display device for displaying the acquired information of the internal structure of the body and information of the specific or critical region or structure and
a storage device for storing the acquired information of the internal structure of the body, information of the specific or critical region or structure and information of the device.

## Patentansprüche

1. Verfahren zur Planung einer Infusion und zur Platzierung eines Katheters in einem Körper, wobei der Katheter eine spezifische Steifigkeit aufweist, die aus verschiedenen, in einer Datenbank bereitgestellten Kathetertypen gewählt wird, wobei Informationen über wenigstens einen Teil der Innenstruktur des Patientenkörpers unter Verwendung von Segmentierungstechniken automatisch analysiert werden, um zu ermitteln, ob zumindest eine spezifische oder kritische Region oder Struktur innerhalb eines interessierenden Bereiches um die geplante Trajektorie der Vorrichtung im Körper liegt, wobei diese spezifische oder kritische Region oder Struktur nach vorgegebenen Risikograden unter Verwendung von in Datenbanken gespeicherten Werten automatisch angezeigt und/oder eingestuft und/oder bewertet wird, wobei die Risikograde und die Kathetersteifigkeit basierend auf dem Risiko aufgrund der patientenspezifischen Gewebedichte einer spezifischen Region oder Struktur des Körpers ermittelt werden.

2. Verfahren nach dem vorherigen Anspruch, wobei anatomische, funktionelle, angiographische und/oder physiologische Körperstrukturen insbesondere automatisch erfasst werden und/oder manuell umrissene Strukturen erfasst werden als Informationen über wenigstens einen Teil der Innenstruktur des Patientenkörpers.

3. Verfahren nach einem der vorherigen Ansprüche, wobei die anatomischen Strukturen durch Kernspinbildgebung (MRI = magnetic resonance imaging), Röntgenbildgebung, Computertomographie-(CT)-Bildgebung, Ultraschallbildgebung gewonnen werden, die physiologischen Strukturen durch MR-DTI, MR-DCE, Perfusionsbildgebung aus MR oder CT, PET, SPECT, MEG ermittelt werden, und die funktionellen Strukturen durch fMRI, PET, Brain-Mapping, EEG ermittelt werden.

4. Verfahren nach einem der vorherigen Ansprüche, wobei der interessierende Bereich um die Trajektorie durch wenigstens einen Voreinstellungs- oder vorgegebenen Wert nahe der Trajektorie oder um die Trajektorie definierbar und/oder von einem Benutzer auswählbar und/oder unter Verwendung von Informationen über den Patienten oder über die Behandlung des Patienten halbautomatisch oder automatisch definierbar ist.

5. Verfahren nach einem der vorherigen Ansprüche, wobei die spezifische oder kritische Region oder Struktur im interessierenden Bereich ferner nach räumlicher Lage, potenzieller negativer Auswirkung und/oder Näherung an die Trajektorie automatisch angezeigt und/oder eingestuft und/oder bewertet wird.

6. Verfahren nach einem der vorherigen Ansprüche, wobei die spezifische oder kritische Region oder Struktur in den Informationen über wenigstens einen Teil der Innenstruktur des Körpers identifiziert und aus den Informationen über die Innenstruktur des Körpers segmentiert wird.

7. Verfahren nach einem der vorherigen Ansprüche, wobei die spezifische oder kritische Region oder Struktur beispielsweise durch MRI-Diffusionsscans, MRI-kontrastverschärfte Scans, DTI, dynamisch MRI-kontrastverschärfte Scans, CT-Perfusionsscans, MRI-T1w-Scans, MRI-T2w-Scans, MRI-Protonendichte-Scans, MRI-Spektroskopie-Scans, PET-Scans, SPECT-Scans, molekulare Bildgebung, CT, Röntgen, Ultraschall, Elastographie, Zeitreihenbildgebung, Biopsien und/oder Gewebeanalyse gewonnen wird.

8. Verfahren nach einem der vorherigen Ansprüche, wobei die segmentierte spezifische oder kritische Region oder Struktur mit einem Abbild des Teils der Innenstruktur des Körpers registriert und diesem Abbild überlagert wird.

9. Verfahren nach dem vorherigen Anspruch, wobei die spezifische oder kritische Region oder Struktur zusammen mit der geplanten Trajektorie auf dem Abbild der Innenstruktur des Körpers angezeigt wird.

10. Verfahren nach einem der vorherigen Ansprüche, wobei die Risikograde ermittelt werden, und zwar basierend auf:
- dem Risiko aufgrund von anatomischen und/oder funktionellen und/oder physiologischen Eigenschaften einer spezifischen Region oder Struktur des Körpers, insbesondere den Risiken einer Schädigung des Körpers und/oder dem Risiko einer erfolglosen Therapie; und/oder
- dem Risiko aufgrund eines vorgegebenen Behandlungsplans, insbesondere aufgrund der Näherung eines Behandlungsansatzes, wie etwa einer Trajektorie, an eine Risikostruktur, und/oder aufgrund der Näherung zweier oder mehrerer verschiedener Behandlungsansätze, wie etwa zweier oder mehrerer Trajektorien, aneinander und/oder basierend auf einer Gewichtung der Gefährlichkeit des vorgegebenen Behandlungsansatzes; und/oder
- dem Risiko aufgrund eines vorgegebenen Setups oder einer vorgegebenen Konfiguration eines chirurgischen Navigationssystems.

11. Verfahren nach dem vorherigen Anspruch, wobei das Risiko einer Schädigung des Körpers das Risiko einer Überquerung einer Trajektorie und eines optischen Nervs umfasst und das Risiko einer erfolglosen Behandlung das Risiko einer Nichtüberquerung der Trajektorie durch einen Sulcus bei der Medikamentenverabreichung umfasst.

12. Verfahren nach einem der beiden vorherigen Ansprüche, wobei die Gewichtung der Gefährlichkeit des vorgegebenen Behandlungsansatzes die Gewichtung der Gefährlichkeit eines Katheterplanes oder einer Kathetertrajektorie umfasst, der bzw. die wenigstens einen vorgegebenen Abstand von beispielsweise einem Sulcus aufweist.

13. Verfahren nach einem der drei vorherigen Ansprüche, wobei das Risiko aufgrund eines vorgegebenen Setups oder einer vorgegebenen Konfiguration des chirurgischen Navigationssystems das Risiko der Verwendung einer nichtmontierten Haltevorrichtung, wie eines Pointers, zur Führung eines Katheters entlang der Trajektorie und/oder das Risiko der Verwendung einer flexiblen Infusions- oder Haltevorrichtung und/oder das Risiko einer weit von einer Eintrittstelle des Katheters entfernt fixierten oder montierten Haltevorrichtung und/oder das Risiko eines unerfahrenen Chirurgen umfasst.

14. Verfahren nach einem der vorherigen Ansprüche, wobei Risikograde separat und/oder hervorgehoben, zum Beispiel farbig, in der spezifischen oder kritischen Region oder Struktur angezeigt werden.

15. Verfahren nach einem der vorherigen Ansprüche, wobei Warnhinweise über die potenzielle negative Auswirkung, wie etwa die Überquerung der Trajektorie und der spezifischen oder kritischen Region oder Struktur, angezeigt werden.

16. Verfahren nach einem der vorherigen Ansprüche, wobei quantitative Informationen bezüglich der Näherung der spezifischen oder kritischen Region oder Struktur in Bezug auf eine oder mehrere Trajektorien bereitgestellt werden.

17. Verfahren nach einem der vorherigen Ansprüche, wobei Grenzen entlang der oder um die spezifische oder kritische Region oder Struktur angezeigt werden, wobei, wenn bei einer Planung die Trajektorie wenigstens eine Grenze überquert, ein Warnsignal - insbesondere ein akustisches Warnsignal - abgegeben wird oder eine Überquerung der Grenze durch die Trajektorie verhindert wird.

18. Verfahren nach einem der vorherigen Ansprüche, wobei Trajektorien ermittelt werden, die Regionen eines vorgegebenen oder vorbestimmten Risikogrades überqueren oder nicht überqueren, insbesondere Trajektorien, die in einem vorbestimmten Abstand zu Bereichen eines vorgegebenen oder vorbestimmten Risikogrades platziert sind.

19. Verfahren nach einem der vorherigen Ansprüche, wobei die geplante Trajektorie für stereotaktische Biopsien, die Katheter-Platzierung, die Kanülen-Platzierung, eine Stimulator-Platzierung, eine Shunt-Platzierung, eine Platzierung chirurgischer Implantate und/oder die Platzierung anderer Vorrichtungen benutzt wird.

20. Verfahren nach einem der vorherigen Ansprüche, wobei bei einer robotischen oder halbrobotischen Vorrichtung, insbesondere einem Roboterarm oder einer Führungsvorrichtung, eine haptische Rückmeldung bereitgestellt wird, wodurch die haptische Rückmeldung auf der Näherung oder Nähe zwischen einem chirurgischen Werkzeug, das sich in einem bekannten Verhältnis zum Patienten bewegt, und Risikobereichen basiert.

21. Computerprogramm, das, wenn es auf einem Computer läuft oder in einen Computer geladen ist, den Computer veranlasst, ein Verfahren gemäß einem der vorherigen Ansprüche durchzuführen.

22. Computerprogrammprodukt, das auf einem computerkompatiblen Medium oder Datenträger gespeichert ist und das Computerprogramm nach dem vorherigen Anspruch aufweist.

23. Vorrichtung zur Planung einer Infusion und zur Platzierung eines Katheters in einem Körper, wobei der Katheter eine spezifische Steifigkeit aufweist, die aus verschiedenen, in einer Datenbank bereitgestellten Kathetertypen gewählt wird, mit:
- eine Akquirierungsvorrichtung zur Akquirierung oder Aufnahme von Informationen über wenigstens einen Teil der Innenstruktur des Patientenkörpers;
- eine Datenbank, die Werte für die Ermittlung von Risikograden speichert;
- eine Analysevorrichtung zur Analyse der akquirierten Informationen über die Innenstruktur des Körpers unter Verwendung von Segmentierungstechniken, um zu ermitteln, ob zumindest eine spezifische oder kritische Region oder Struktur innerhalb eines interessierenden Bereiches um die geplante Trajektorie der Vorrichtung im Körper liegt, und um diese spezifische oder kritische Region oder Struktur nach vorgegebenen Risikograden unter Verwendung der in der Datenbank gespeicherten Werte anzuzeigen und/oder einzustufen und/oder zu bewerten, wobei die Risikograde und die Kathetersteifigkeit basierend auf dem Risiko aufgrund der patientenspezifischen Gewebedichte einer spezifischen Region oder Struktur des Körpers ermittelt werden.

24. Vorrichtung nach Anspruch 23, die außerdem Folgendes aufweist:
- eine Anzeigevorrichtung zur Anzeige der akquirierten Informationen über die Innenstruktur des Körpers und zur Anzeige von Informationen über die spezifische oder kritische Region oder Struktur; und
- eine Speichervorrichtung zur Speicherung der akquirierten Informationen über die Innenstruktur des Körpers, zur Speicherung von Informationen über die spezifische oder kritische Region oder Struktur, und zur Speicherung von Informationen über die Vorrichtung.

## Revendications

1. Procédé pour préparer une perfusion et le placement d'un cathéter ayant une rigidité spécifique, choisi parmi plusieurs types de cathéters contenus dans une base de données, dans un corps, dans lequel des informations d'au moins une partie de la structure interne du corps d'un patient sont automatiquement analysées en utilisant des techniques de segmentation pour déterminer si au moins une région ou structure spécifique ou critique est contenue dans une région d'intérêt autour de la trajectoire prévue du dispositif dans le corps, dans lequel ladite région ou structure spécifique ou critique est automatiquement affichée et/ou classée et/ou évaluée en termes de niveaux de risque prédéfinis en utilisant des valeurs mémorisées dans des bases de données, dans lequel les niveaux de risque et la rigidité du cathéter sont déterminés sur la base du risque dû à la densité de tissu spécifique au patient d'une région ou structure spécifique du corps.

2. Procédé selon la revendication précédente, dans lequel en tant qu'informations d'au moins une partie de la structure interne du corps, des structures anatomiques, fonctionnelles, angiographiques et/ou physiologiques du corps sont, en particulier automatiquement, détectées et/ou des structures tracées manuellement sont détectées.

3. Procédé selon l'une quelconque des revendications précédentes, dans lequel les structures anatomiques sont obtenues en utilisant une imagerie par résonance magnétique (IRM), une imagerie par rayons X, une tomographie informatique (CT), une imagerie par ultrasons, les structures physiologiques sont déterminées en utilisant MR-DTI, MR-DCE, une imagerie par perfusion à partir de MR ou CT, PET, SPECT, MEG, et les structures fonctionnelles sont déterminées en utilisant fIRM, PET, le mappage du cerveau, EEG.

4. Procédé selon l'une quelconque des revendications précédentes, dans lequel la région d'intérêt autour de la trajectoire peut être définie par au moins une valeur par défaut ou prédéterminée proche de la trajectoire ou autour de la trajectoire et/ou peut être sélectionnée par un utilisateur et/ou peut être semi-automatiquement ou automatiquement définie en utilisant des informations concernant le patient ou le traitement du patient.

5. Procédé selon l'une quelconque des revendications précédentes, dans lequel la région ou structure spécifique ou critique dans la région d'intérêt est en outre automatiquement affichée et/ou classée et/ou évaluée en termes de position spatiale, d'effet indésirable potentiel et/ou de proximité par rapport à la trajectoire.

6. Procédé selon l'une quelconque des revendications précédentes, dans lequel la région ou structure spécifique ou critique est identifiée dans les informations d'au moins une partie de la structure interne du corps et est segmentée à partir des informations de la structure interne du corps.

7. Procédé selon l'une quelconque des revendications précédentes, dans lequel la région ou structure spécifique ou critique est obtenue par exemple par balayages IRM de diffusion, balayages IRM rehaussés par un agent de contraste, DTI, balayages IRM rehaussés par un agent de contraste dynamique, balayages IRM à densité de protons, balayages IRM spectroscopiques, balayages PET, balayages SPECT, imagerie moléculaire, CT, rayons X, ultrasons, élastographie, imagerie chronologique, biopsies et/ou analyse de tissu.

8. Procédé selon l'une quelconque des revendications précédentes, dans lequel la région ou structure spécifique ou critique est enregistrée avec une image de la partie de la structure interne du corps et est superposée sur ladite image.

9. Procédé selon la revendication précédente, dans lequel la région ou structure spécifique ou critique est affichée conjointement avec la trajectoire prévue sur l'image de la structure interne du corps.

10. Procédé selon l'une quelconque des revendications précédentes, dans lequel les niveaux de risque sont déterminés sut la base :
d'un risque dû aux caractéristiques anatomiques et/ou fonctionnelles et/ou physiologiques d'une région ou structure spécifique du corps, en particulier des risques de blessure du corps et/ou un risque d'échec de la thérapie, et/ou
d'un risque dû à un plan de traitement prédéfini, en particulier la proximité d'une approche de traitement, telle qu'une trajectoire, par rapport à une structure de risque, et/ou la proximité de deux ou plus de deux approches de traitement différentes, telles que deux ou plus de deux trajectoires, les unes par rapport aux autres et/ou la pondération des risques de l'approche de traitement prédéfinie, et/ou
d'un risque dû à un paramétrage ou à une configuration prédéfini d'un système de navigation chirurgical.

11. Procédé selon la revendication précédente, dans lequel le risque de blessure du corps comporte le risque de traverser une trajectoire et un nerf optique et le risque d'échec du traitement comporte le risque de ne pas traverser la trajectoire via un sillon lors de l'administration de médicament.

12. Procédé selon l'une quelconque des revendications précédentes, dans lequel la pondération des risques de l'approche de traitement prédéfinie comporte la pondération des risques d'un plan ou d'une trajectoire de cathéter qui est au moins éloigné d'une distance prédéterminée par exemple d'un sillon.

13. Procédé selon l'une quelconque des trois revendications précédentes, dans lequel le risque dû à un paramétrage ou à une configuration prédéfini du système de navigation chirurgical comporte un risque d'utilisation d'un dispositif de maintien non monté, tel qu'une aiguille pour faire passer un cathéter le long de la trajectoire et/ou le risque d'une utilisation d'une perfusion flexible ou d'un dispositif de maintien et/ou le risque lorsque le dispositif de maintien est fixé ou monté loin d'un point d'entrée du cathéter et/ou le risque d'un chirurgien inexpérimenté.

14. Procédé selon l'une quelconque des revendications précédentes, dans lequel des niveaux de risque sont affichés séparément et/ou surlignés, par exemple colorés, dans la région ou structure spécifique ou critique.

15. Procédé selon l'une quelconque des revendications précédentes, dans lequel des avertissements concernant l'effet indésirable potentiel, tel que le croisement de la trajectoire et de la région ou structure spécifique ou critique, sont affichés.

16. Procédé selon l'une quelconque des revendications précédentes, dans lequel des informations quantitatives concernant la proximité de la région ou structure spécifique ou critique par rapport à une ou plusieurs trajectoires sont fournies.

17. Procédé selon l'une quelconque des revendications précédentes, dans lequel des bords sont affichés le long ou autour de la région ou structure spécifique ou critique, dans lequel lorsqu'il est prévu que la trajectoire traverse au moins un bord, un signal d'avertissement, en particulier un signal d'avertissement acoustique, est délivré en sortie ou on empêche la trajectoire de traverser le bord.

18. Procédé selon l'une quelconque des revendications précédentes, dans lequel sont déterminées des trajectoires qui traversent ou ne traversent pas des régions ayant un niveau de risque prédéfini ou prédéterminé, en particulier qui sont situées à une distance prédéterminée de zones ayant un niveau de risque prédéfini ou prédéterminé.

19. Procédé selon l'une quelconque des revendications précédentes, dans lequel la trajectoire prévue est utilisée pour des biopsies stéréotaxiques, un placement de cathéter, un placement de canule, un placement de stimulateur, un placement de dérivation, un placement d'implants chirurgicaux et/ou un placement d'autres dispositifs.

20. Procédé selon l'une quelconque des revendications précédentes, dans lequel une réaction haptique est délivrée à un dispositif robotisé ou semi-robotisé, en particulier un bras robotisé ou un dispositif de guidage, la réaction haptique étant basée sur la proximité entre un instrument chirurgical se déplaçant selon une relation connue dans le patient et des régions de risque.

21. Programme informatique lequel, lorsqu'il est exécuté sur un ordinateur ou chargé sur un ordinateur, amène l'ordinateur à mettre en oeuvre un procédé selon l'une quelconque des revendications précédentes.

22. Produit de programme informatique mémorisé sur un support compatible avec un ordinateur ou un support de données et comportant le programme informatique tel que défini dans la revendication précédente.

23. Dispositif pour préparer une perfusion et le placement d'un cathéter ayant une rigidité spécifique, choisi parmi plusieurs types de cathéters contenus dans une base de données, dans un corps, comportant :
un dispositif d'acquisition pour acquérir des informations d'au moins une partie de la structure interne du corps d'un patient
une base de données mémorisant des valeurs pour la détermination de niveaux de risque
un dispositif d'analyse pour analyser les informations acquises de la structure interne du corps en utilisant des techniques de segmentation pour déterminer si au moins une région ou structure spécifique ou critique est contenue dans une région d'intérêt autour de la trajectoire prévue du dispositif dans le corps et pour afficher et/ou classer et/ou évaluer ladite région ou structure spécifique ou critique en termes de niveaux de risque prédéfinis en utilisant les valeurs mémorisées dans la base de données, dans lequel les niveaux de risque et la rigidité du cathéter sont déterminés sur la base du risque dû à la densité de tissu spécifique au patient d'une région ou structure spécifique du corps.

24. Dispositif selon la revendication 23, comportant en outre
un dispositif d'affichage pour afficher les informations acquises de la structure interne du corps et des informations de la région ou structure spécifique ou critique et
un dispositif de mémorisation pour mémoriser les informations acquises de la structure interne du corps, des informations de la région ou structure spécifique ou critique et des informations du dispositif.
